(19) Europäisches Patentamt
European Patent Office
Office européen des brevets

(11) Veröffentlichungsnummer: **0 034 759**
**B1**

(12) # EUROPÄISCHE PATENTSCHRIFT

(45) Veröffentlichungstag der Patentschrift: **02.01.86**

(51) Int. Cl.⁴: **C 07 D501/36**, C 12 Q 1/34

(21) Anmeldenummer: **81100959.6**

(22) Anmeldetag: **11.02.81**

(54) **Chromogene Cephalosporine und Verfahren zu ihrer Herstellung.**

(30) Priorität: **23.02.80 DE 3006889**

(43) Veröffentlichungstag der Anmeldung: **02.09.81 Patentblatt 81/35**

(45) Bekanntmachung des Hinweises auf die Patenterteilung: **02.01.86 Patentblatt 86/01**

(84) Benannte Vertragsstaaten: **DE FR GB IT**

(56) Entgegenhaltungen:
**EP-A- 0 018 001**
**US-A- 3 278 531**
**US-A- 3 830 700**

(73) Patentinhaber: **HOECHST AKTIENGESELLSCHAFT**
**Postfach 80 03 20**
**D-6230 Frankfurt am Main 80 (DE)**

(72) Erfinder: **Blumbach, Jürgen, Dr.**
**Merziger Weg 1a**
**D-6000 Frankfurt am Main 71 (DE)**
Erfinder: **Schindler, Peter, Dr.**
**Reinhardswaldweg 1**
**D-6082 Mörfelden-Walldorf (DE)**

**EP 0 034 759 B1**

Anmerkung: Innerhalb von neun Monaten nach der Bekanntmachung des Hinweises auf die Erteilung des europäischen Fatents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Der Einsatz von β-Lactam-Antibiotika für die Chemotherapie bakterieller Infektionen wird eingeschränkt durch das Auftreten β-Lactam-resistenter Keime, die β-Lactamasen (Penicillin-(Cephalosporin)-β-Lactam-Amidohydrolase, EC 3.5.2.6) produzieren. Diese Enzyme spalten die C-N-Bindung des β-Lactamrings, wodurch die antibiotischen Eigenschaften der eingesetzten β-Lactame verloren gehen. Um den gewünschten Therapieerfolg bei der Behandlung bakterieller Infektionen mit β-Lactam-Antibiotika zu sichern, müssen vor Therapiebeginn gesicherte Erkenntnisse darüber vorliegen, ob die klinischen Isolate β-Lactamasen produzieren oder nicht. Fällt der β-Lactamase-Nachweis positiv aus, empfiehlt sich entweder der Einsatz von β-Lactamase-resistenten β-Lactam-Antibiotika oder aber von anderen klinisch anwendbaren Antibiotika.

Für den Nachweis von β-Lactamase-produzierenden Keimen sind eine Reihe von Techniken beschrieben worden (vgl. J. Pharmacol. *15* (1963), S. 81-91). Die genannten Methoden sind jedoch alle nicht völlig befriedigend, da sie, sofern es sich um die für klinisch-diagnostische Zwecke besonders geeigneten colorometrischen Teste handelt, alle den Zusatz eines Indikators (Jod/Stärke, Hydroxylamin, pH-Indikator) benötigen.

Chromogene β-Lactamase-Substrate bieten gegenüber β-Lactamase-Nachweisen mit Hilfe eines Indikator-gekoppelten Testansatzes den Vorteil, daß die Öffnung des β-Lactamrings durch diese Enzyme unmittelbar zu einer Farbverschiebung im sichtbaren Teil des Spektrums führt. Von O'Callaghan et al. (Antimicrob. Ag. Chemother. *1* (1972), S. 283-288) wurde ein solches chromogenes β-Lactamase-Substrat (Verbindung 87/312) beschrieben. Nach Angaben der Autoren tritt jedoch die bathochrome Verschiebung der Farbe von Lösungen dieser Verbindung nicht nur in Gegenwart von β-Lactamasen ein, sondern in unspezifischer Weise auch nach Zusatz von Serum, Tiergewebe, Eiweiß, Milch, Cystein, Glutathion, Mercaptoäthanol und 2,3-Dimercaptopropanol-1. Die Verwendung der Verbindung 87/312 kann daher zu falsch-positiven Beurteilungen klinischer Isolate führen.

Gegenstand der vorliegenden Erfindung sind deshalb neue chromogene Cephalosporine, die durch eine optisch gut sichtbare Farbvertiefung von leicht gelb nach intensiv gelb-orange die Gegenwart von β-Lactamasen anzeigen und gegen die obengenannten Zusätze stabil sind. Der Nachweis von β-Lactamasen kann sowohl mit intakten Zellen β-Lactamase-produzierender Keime als auch mit den aus diesen Organismen mit Hilfe von Standard-Methoden isolierten Enzymen gleichermaßen gut durchgeführt werden. Über den reinen Nachweis hinaus sind die erfindungsgemäßen Verbindungen auch für die Bestimmung der spezifischen Aktivität isolierter β-Lactamasen sehr gut geeignet.

Die erfindungsgemäßen Verbindungen werden von allen untersuchten β-Lactamasen aus gramnegativen Organismen unter Bildung der oben angegebenen Farbvertiefung gespalten. Es sind dies insbesondere β-Lactamasen aus klinisch interessanten Organismen, z. B. E. coli $R_{TEM}$, Klebsiella aerogenes 1082 E, Pseudomonas aeruginosa 18 SH, Enterobacter cloacae P99 und Bacteroides fragilis 620. Die erfindungsgemäßen Verbindungen werden ebenso von β-Lactamasen aus gram-positiven Organismen, z. B. Staphylococcus aureus R 85, gespalten.

Der Nachweis der β-Lactamase-Aktivität mit den erfindungsgemäßen Verbindungen erfolgt direkt, d. h. ohne einen gekoppelten Testansatz, da die chromophoren Substituenten, die im obengenannten Zusammenhang Gegenstand der Erfindung sind, synchron mit der Öffnung des β-Lactamrings quantitativ eliminiert werden, was unmittelbar zur oben erwähnten Farbverschiebung führt.

Cephalosporine, die in 3-Stellung eine u. a. durch Nitro, Cyano oder Carboxy substituierte Arylthiomethylgruppe tragen, sind aus U.S.3 278 531 bekannt. Die dort beschriebenen Verbindungen werden als besonders stabil charakterisiert, während gerade die Instabilität unserer erfindungsgemäßen Verbindungen die Grundlage für den mit unserer Erfindung erzielten Farbumschlag darstellt.

Überraschenderweise wurde nun festgestellt, daß die erfindungsgemäßen Verbindungen im Gegensatz zu β-Lactamase-Substraten des Typs 87/312 in wäßrigen Lösungen, die für die Aktivität der β-Lactamasen bzw. für die Lebensfähigkeit von Bakterien geeignet sind, insbesondere aber im neutralen pH-Bereich, außerordentlich beständig sind gegen hydrolytischen Abbau im Temperaturbereich zwischen − 180 °C und 60 °C. Obwohl die Aufbewahrung fertig zubereiteter Lösungen vorteilhafterweise bei − 20 °C erfolgt, bei der die Lösung nahezu unbegrenzt haltbar ist, können neutral gepufferte Lösungen über längere Zeit bei Kühlschranktemperatur und sogar bei Raumtemperatur aufbewahrt werden, ohne daß ihre Verwendbarkeit beeinträchtigt wird.

Darüberhinaus erwiesen sich die erfindungsgemäßen Verbindungen vollkommen stabil gegenüber dem Einfluß von Serum, Eiweiß, Milch, Cystein, Glutathion, Mercaptoäthanol und 2,3-Dimercaptopropanol-1 (Dimercaprol), wodurch eine weitere unerwartete Überlegenheit gegenüber β-Lactamase-Substraten des Typs 87/312 erzielt werden konnte.

Gegenstand der Erfindung sind demnach in 3'-Stellung eine chromogene Gruppierung tragende Cephalosporine der allgemeinen Formel

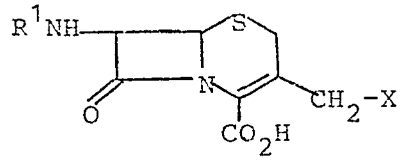

(I)

in der $R^1$ Wasserstoff, Formyl, Benzoyl, das substituiert sein kann durch Alkyl mit 1-4 C-Atomen, durch Halogen, durch Nitro, durch Amino oder durch Acetamido, oder ein Rest der Formel

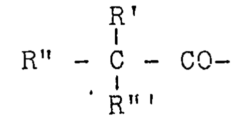

sein kann, in der R' für Wasserstoff, für Alkyl mit 1-4 C-Atomen, das substituiert ist durch Halogen, durch Cyano, durch Hydroxy, durch ω-Carboxy, oder für einen ein- oder mehrfach ungesättigten 5- bis 7-gliedrigen carbocyclischen Ring oder für Phenyl und Phenoxy, die substituiert sind durch Alkyl mit 1-4 C-Atomen, durch Hydroxy, durch Alkoxy mit 1-4 C-Atomen, durch Acyloxy mit 1-4 C-Atomen, durch Halogen, durch Carboxy, durch Sulfoxy, durch Amino oder durch Acylamino mit 1-4 C-Atomen oder für einen 5- oder 6-gliedrigen Heteroaryl- oder Heteroarylthio-Rest, der als Heteroatome im Ring Stickstoff, Schwefel oder Sauerstoff enthalten kann, steht, und in der R'' und R''', die gleich oder verschieden sein können, für Wasserstoff, Hydroxy, Acyloxy mit 1-4 C-Atomen im Acylteil, Amino, Alkylamino, mit 1-6 C-Atomen im Alkylteil, Benzylamino, p-Methoxybenzylamino, Benzhydrylamino, Tritylamino, Phenäthylamino, Formamido, Acetylamino, Chloracetylamino, Bromacetylamino, Benzoylamino, tert.-Butoxycarbonylamino, 2,2,2-Trichloräthoxycarbonylamino, 4-Hydroxy-1,5-naphthyridin-3-carbonylamino, 3-Hydroxy-pyridazin-4-carbonylamino, Imidazolidin-2-on-1-yl-carbonylamino, (3-Methyl-sulfonyl-imidazolidin-2-on-1-yl)-carbonylamino, (4-Äthyl-piperazin-2,3-dion-1-yl)-carbonylamino, Alkoxycarbonyl mit 1-4 C-Atomen mit Alkylteil, Halogen, Cyano, Sulfoxy oder Aminosulfonyl stehen, und X steht für eine Gruppe der Formel

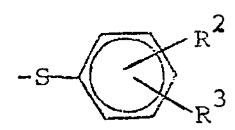

in der $R^2$ für Nitro und $R^3$ für Wasserstoff, Nitro, Halogen, Alkyl mit 1-4 C-Atomen, Cyano, Carboxy, Alkoxycarbonyl mit 1-4 C-Atomen im Alkoxyteil und Aminocarbonyl steht.

Bei den erfindungsgemäßen Verbindungen haben die Substituenten insbesondere die folgenden Bedeutungen :

$R^1$ in der Bedeutung von Benzoyl, kann gegebenenfalls substituiert sein durch Alkyl mit 1-4 C-Atomen, vorzugsweise Methyl, wie z. B. Toluoyl, durch Halogen, vorzugsweise Chlor, wie z. B. p-Chlorbenzoyl, durch eine Nitrogruppe, wie z. B. p-Nitrobenzoyl, durch eine Aminogruppe, wie z. B. p-Aminobenzoyl oder durch eine Acetamidogruppe, wie z. B. p-Acetamidobenzoyl.

R' kann stehen für Wasserstoff, für Alkyl mit 1-4 C-Atomen, vorzugsweise Methyl oder Äthyl, für substituiertes Alkyl mit 1-4 C-Atomen, wie beispielsweise Halogenalkyl mit 1-4 C-Atomen, vorzugsweise Chlormethyl, Brommethyl, Chloräthyl oder Bromäthyl, für Cyanoalkyl mit 1-4 C-Atomen im Alkylteil, vorzugsweise Cyanomethyl oder Cyanoäthyl, für Hydroxyalkyl mit 1-4 C-Atomen, vorzugsweise Hydroxymethyl oder Hydroxyäthyl, für ω-Carboxyalkyl mit 1-4 C-Atomen im Alkylteil, vorzugsweise Carboxymethyl oder Carboxyäthyl, für einen gesättigten oder ein- oder mehrfach ungesättigten 5- bis 7-gliedrigen carbocyclischen Ring, wie Cycloalkyl, vorzugsweise Cyclohexyl, Cycloalkenyl, vorzugsweise Cyclohexenyl, Cycloalkadienyl, vorzugsweise Cyclohexadienyl, für Phenyl oder Phenoxy, die substituiert sind durch $C_1$-$C_4$-Alkyl, vorzugsweise Methyl oder Äthyl, Hydroxy, $C_1$-$C_4$-Alkoxy, vorzugsweise Methoxy oder Äthoxy, $C_1$-$C_4$-Acyloxy, vorzugsweise Acetoxy, Halogen, vorzugsweise Fluor und Chlor, Carboxy, Sulfoxy, Amino, $C_1$-$C_4$-Acylamino, vorzugsweise Acetamino. R' kann weiterhin für 5- bis 6-gliedriges Heteroaryl stehen, das als Heteroatome Stickstoff, Schwefel oder Sauerstoff enthalten kann, wie z. B. 2-Pyridon-1-yl, 4-Pyridon-1-yl, 3,5-Dichlor-4-pyridon-1-yl, 2-Thienyl, 1-Tetrazolyl, vorzugsweise aber 2-Furyl, 2-Thienyl oder 1-Tetrazolyl.

R' kann auch stehen für einen 5- bis 6-gliedrigen Heteroarylthiorest, der als Heteroatome im Ring Stickstoff, Schwefel oder Sauerstoff enthalten kann, wie vorzugsweise 2-Methyl-1,3,4-thiadiazol-5-yl-thio oder 2-Amino-1,3,4-thiadiazol-5-yl-thio.

R'' und R''', die gleich oder verschieden sein können, können die Bedeutung besitzen von Wasserstoff, von Hydroxy, von Acyloxy mit 1-4 C-Atomen im Acylteil, wie z. B. Formyloxy, Acetoxy, Propionyloxy, von Amino, von Alkylamino mit 1-6 C-Atomen im Alkylteil, wie vorzugsweise tert.-Butylamino, tert.-Amylamino, Benzylamino, p-Methoxybenzylamino, Benzhydrylamino, Tritylamino, Phenyläthylamino, von Formamido, Acetylamino, Chloracetylamino, Bromacetylamino, Benzoylamino, tert.-Butoxycarbonylamino, 2,2,2-Trichloräthoxycarbonylamino, 4-Hydroxy-1,5-naphthyridin-3-carbonylamino, 3-Hydroxy-pyridazin-4-carbonylamino, Imidazolidin-2-on-1-yl-carbonylamino, (3-Methyl-sulfonylimidazolidin-2-on-1-yl)-carbonylamino, (4-Äthyl-piperazin-2,3-dion-1-yl)-carbonylamino, von Alkoxycarbonyl mit 1 bis 4 C-Atomen im Alkylteil, wie z. B. Methoxycarbonyl, Äthoxycarbonyl, von Halogen, vorzugsweise Chlor und Brom, von Cyano, von Sulfoxy oder von Aminosulfonyl.

In dem Rest X der Formel

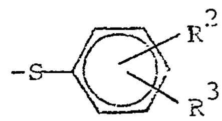

kann $R^2$ insbesondere für eine Nitrogruppe in para- oder ortho-Stellung zum Schwefel, und $R^3$ für Wasserstoff, eine Nitrogruppe, Halogen, Alkyl mit 1-4 C-Atomen, wie vorzugsweise Methyl, Äthyl, n-Propyl, i-Propyl, n-Butyl und tert.-Butyl, für Cyano, Carboxy, für Alkoxycarbonyl mit 1-4 C-Atomen im Alkylteil, wie vorzugsweise Methoxycarbonyl und Äthoxycarbonyl und für Aminocarbonyl stehen.

Besonders bevorzugte Bedeutungen von X sind p-Nitrophenylthio, o-Nitrophenylthio, o,p-Dinitrophenylthio, m-Carboxy-p-nitrophenylthio, m-Cyano-p-nitrophenylthio, o-Cyano-p-nitrophenylthio, p-Carboxy-o-nitrophenylthio und p-Cyano-o-nitrophenylthio.

Gegenstand der Erfindung ist weiterhin ein Verfahren zur Herstellung von Cephalosporinen der allgemeinen Formel I, das dadurch gekennzeichnet ist, daß man eine Verbindung der Formel III

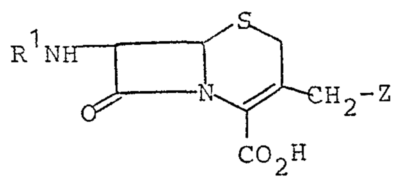

(III)

in der Z Formyloxy, Acetoxy, Acetoacetoxy oder Aminocarbonyloxy sein kann, und $R^1$ die vorgenannte Bedeutung hat, mit einer Verbindung der Formel H—X, in der X die obengenannte Bedeutung hat, umsetzt und im Falle, daß $R^1$ Wasserstoff bedeutet, mit Ameisensäure, mit einer Phenylcarbonsäure, die im Phenylrest durch $C_1$-$C_4$-Alkyl, Halogen, Nitro, Amino oder Acetamido substituiert sein kann, oder mit einer Carbonsäure der Formel IV

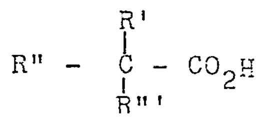

(IV)

in der R′, R″ und R‴ die vorstehend genannten Bedeutungen haben, oder einem reaktionsfähigen Derivat derselben zur Umsetzung bringt.

Die zur Durchführung des erfindungsgemäßen Verfahrens erforderlichen Ausgangssubstanzen sind literaturbekannt oder können nach literaturbekannten Verfahren hergestellt werden.

Die Umsetzung der Verbindung III mit Verbindungen der Formel H—X wird zweckmäßigerweise in wäßrigem Medium durchgeführt, wobei zur Verbesserung der Löslichkeit der an der Reaktion beteiligten Komponenten mit Wasser mischbare organische Lösungsmittel zugegeben werden können, wie vorzugsweise Methanol, Äthanol, Isopropanol, Acetonitril, Aceton, Dimethylformamid, Dimethylacetamid, Tetrahydrofuran oder Dioxan.

Die Verbindung der Formel H—X kann auch in Form eines Salzes, beispielsweise eines Alkali- oder Erdalkalisalzes, insbesondere eines Natrium- oder Kaliumsalzes, oder eines Salzes mit einem gegebenenfalls substituierten Ammoniumkation eingesetzt werden.

Die Reaktionskomponenten werden in beliebigem Verhältnis, vorzugsweise jedoch in molarem bis etwa 5-molarem Verhältnis umgesetzt.

Die Temperatur der Reaktion ist nicht kritisch, sollte jedoch vorzugsweise bei etwa 20 bis 80 °C liegen. Es kann vorteilhaft sein, dem Reaktionsgemisch ein bei der gewünschten Reaktionstemperatur siedendes organisches, in Wasser lösliches oder unlösliches Lösungsmittel zuzugeben, wodurch die Reaktionstemperatur konstant gehalten wird.

Während der Reaktion wird der pH-Wert des Reaktionsgemisches durch Zugabe von Säure oder Base in einem Bereich von etwa 5-8, vorzugsweise jedoch zwischen 6,5 und 7,5 gehalten.

In einer anderen Ausführungsform können die erfindungsgemäßen Verbindungen so hergestellt werden, daß man ein Cephalosporin der Formel I, in der $R^1$ für Wasserstoff steht und Z die obengenannte Bedeutung hat, mit Ameisensäure, mit einer Phenylcarbonsäure, die wie vorstehend angegeben substituiert sein kann, oder mit einer Carbonsäure der Formel IV

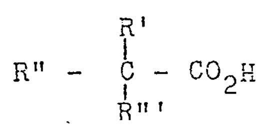

(IV)

in der R′, R″ und R‴ die vorgenannten Bedeutungen haben, oder einem aktivierten Derivat derselben in an sich bekannter Weise umsetzt.

Als aktivierte Derivate eignen sich insbesondere die Halogenide, vorzugsweise Chloride und Bromide, ferner die Anhydride und gemischten Anhydride, die Azide und aktivierten Ester, vorzugsweise die mit p-Nitrophenol, 2,4-Dinitrophenol, Methylencyanhydrin, N-Hydroxysuccinimid und N-Hydroxyphthalimid, besonders bevorzugt mit 1-Hydroxybenzotriazol und 6-Chlor-1-H-hydroxybenzotriazol. Als gemischte Anhydride eignen sich besonders solche mit niederen Alkansäuren, z. B. mit Essigsäure und besonders bevorzugt mit substituierten Essigsäuren, wie z. B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäuren der Formel IV mit Chlorameisensäurebenzylester, -p-nitrobenzylester, -iso-butylester, -äthylester oder -allylester gewinnt. Die aktivierten Derivate können als isolierte Substanzen, aber auch in situ umgesetzt werden. Allgemein erfolgt die Umsetzung der Cephemderivate I, worin $R^1$ für Wasserstoff steht, mit der Carbonsäure IV oder einem aktivierten Derivat derselben in Gegenwart eines inerten Lösungsmittels. Insbesondere eignen sich chlorierte Kohlenwasserstoffe, wie vorzugsweise Methylenchlorid und Chloroform ; Äther, wie z. B. Diäthyläther, Diisopropyläther und vorzugsweise Tetrahydrofuran und Dioxan ; Ketone, wie vorzugsweise Aceton und Butanon ; Amide, wie vorzugsweise Dimethylformamid und Dimethylacetamid oder Wasser. Es kann sich auch als vorteilhaft erweisen, Gemische der genannten Lösungsmittel zu verwenden. Dies ist oftmals der Fall, wenn die Cephemverbindung I, worin $R^1$ für Wasserstoff steht, mit einem in situ erzeugten aktivierten Derivat der Carbonsäure IV umgesetzt wird.

Die Umsetzung von Cephemverbindungen I, worin $R^1$ für Wasserstoff steht mit den vorstehend genannten Carbonsäuren bzw. deren aktivierten Derivaten kann in einem Temperaturbereich von etwa − 50 bis etwa + 80 °C, vorzugsweise zwischen − 20 und + 50 °C, besonders bevorzugt jedoch zwischen − 20 °C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

Die Umsetzung von aktivierten Derivaten der Carbonsäuren mit Cephemverbindungen der Formel I, worin $R^1$ für Wasserstoff steht, wird vorzugsweise in alkalischem Milieu oberhalb pH 7 vorgenommen. Man setzt hierfür dem Reaktionsgemisch eine Base zu, wie vorzugsweise Kalium- oder Natriumcarbonat, Kalium- oder Natriumbicarbonat, Kalium- oder Natriumhydroxy, Pyridin oder ein Trialkylamin, wie z. B. Triäthylamin, N-Methylmorpholin, Äthyldiisopropylamin oder Kalium-tert.-butylat.

Die erfindungsgemäßen Verbindungen der Formel I können isoliert werden, indem nach beendeter Umsetzung die zugesetzten organischen Lösungsmittel abdestilliert werden, die wäßrige Phase zur Entfernung z. B. nicht-umgesetzter Austauschkomponenten der Formel H—X mit Essigester oder einem anderen geeigneten Lösungsmittel extrahiert wird und die wäßrige Phase anschließend auf einen pH von 2-4, vorzugsweise von 3 angesäuert wird, wobei die Verbindungen I ausfallen und beispielsweise durch Filtration abgetrennt werden können.

In einer anderen Ausführungsform zur Isolierung der erfindungsgemäßen Verbindungen kann die wäßrige Phase bei pH 2-4, vorzugsweise bei pH 3 mit einem mit Wasser nicht mischbaren Lösungsmittel wie vorzugsweise Essigester extrahiert werden. Nach Einengen des Extraktes im Vakuum werden die Reaktionsprodukte durch Zusatz eines organischen Lösungsmittels, in welchem diese schwer löslich sind, beispielsweise Äther oder Petroläther, ausgefällt.

In einer weiteren Ausführungsform können die Reaktionsprodukte der Formel I auch bei höheren pH-Werten als pH 4 direkt aus dem Reaktionsansatz in ein mit Wasser nicht mischbares organisches Lösungsmittel, wie vorzugsweise Essigester, extrahiert werden. Die weitere Aufarbeitung gestaltet sich dann wie oben beschrieben.

Außer den in den Ausführungsbeispielen genannten Verbindungen lassen sich erfindungsgemäß beispielsweise auch die in der nachfolgenden Tabelle aufgeführten Verbindungen der Formel I herstellen.

| $R^1$ | X | $R^1$ | X |
|---|---|---|---|
| $CH_3CO$ | 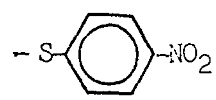 | HCO | 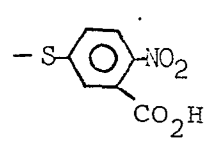 |
| $C_6H_5-CO$ | " | $CH_3CO$ | " |
| $CH_3CO$ | 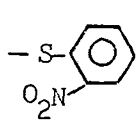 | $C_6H_5CO$ | " |
| HCO | " | | |

(Fortsetzung)

| $R^1$ | X | $R^1$ | X |
|---|---|---|---|
| $C_6H_5CO$ | " | HCO | 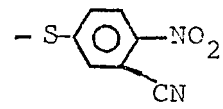 |
| 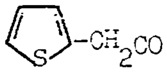 | " | $CH_3CO$ | " |
| HCO | 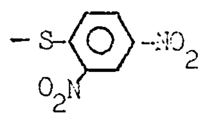 | $C_6H_5CO$ | " |
| $CH_3CO$ | " | 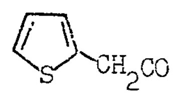 | 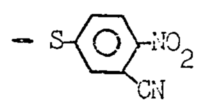 |
| $C_6H_5CO$ | " | HCO | 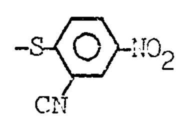 |
| 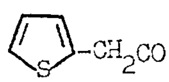 | " | $CH_3CO$ | " |
| HCO | 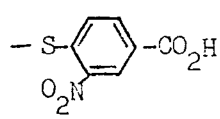 | $C_6H_5CO$ | " |
| $CH_3CO$ | " | 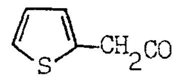 | " |
| $C_6H_5CO$ | " | $CH_3CO$ | 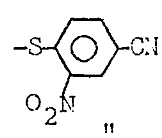 |
| 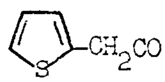 | " | $C_6H_5CO$ | " |
| HCO | 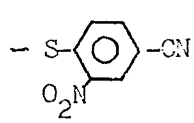 | 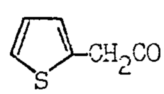 | " |

Die Beispiele erläutern die Erfindung, ohne sie jedoch einzuschränken.

Die für den allgemeinen Nachweis von β-Lactamasen besonders bevorzugte 7-β-Thien-2-yl-acetaido-3-[(4-nitro-3-carboxyphenyl)-thiomethyl]-3-cephem-4-carbonsäure ist in gepufferten Lösungen (pH 7,3) schwach gelb gefärbt ($\lambda_{max}$ 344 nm). Nach Reaktion mit einer β-Lactamase, z. B. dem Enzym aus Enterobacter cloacae P99, ensteht die für p-Nitrothiophenolate charakteristische Gelbfärbung ($\lambda_{max}$ 405 nm). Die Intensität der Farbe ist naturgemäß vom pH-Wert der Lösung abhängig und erreicht bei pH 8,0 ihr Maximum. Für Routine-Bestimmungen ist jedoch ein pH-Wert von 7,3 als ausreichend anzusehen. Der größte ausnutzbare Extinktionsunterschied von Substrat und Reaktionsprodukt liegt bei 415 nm. Eine analoge Reaktion dieser bevorzugten Verbindung erfolgt auch mit anderen β-Lactamasen, z. B. aus Escherichia coli $R_{TEM}$, Klebsiella aerogenes 1082 E, Pseudomonas aeruginosa 18 SH, Bacteroides fragilis 620 und Staphylococcus aureus R 85. Andere erfindungsgemäße chromogene Cephalosporine ergeben bei der Umsetzung mit β-Lactamasen entsprechende Farbänderungen.

Die erfindungsgemäßen Verbindungen werden zum Nachweis von β-Lactamasen verwendet, indem z. B. neutral gepufferte wäßrige Lösungen der chromogenen Cephalosporine direkt mit isolierten β-Lactamasen oder aber mit Zellsuspensionen von β-Lactamase-produzierenden Keimen versetzt werden,

wobei bei Wahl geeigneter Konzentration des zur Anwendung kommenden chromogenen Cephalosporins die charakteristische Farbentwicklung beobachtet wird.

Alternativ können anderweitig gewonnene klinische Isolate in Form eines Kolonie-Abstrichs auf angefeuchtete Teststreifen aus Papier oder einem anderen geeigneten Träger-Material, z. B. saugfähige Kunststoffe, Dextrane oder andere natürliche Polymere, ausgestrichen werden, die vorher mit geeigneten, vorzugsweise wäßrigen Lösungen der erfindungsgemäßen Verbindungen getränkt wurden. Auch hier zeigen β-Lactamase-positive Proben innerhalb kurzer Zeit den charakteristischen Farbumschlag.

Darüberhinaus können die erfindungsgemäßen Verbindungen bei der Reinigung von β-Lactamasen verwendet werden, um z. B. bei säulenchromatographischen Trennverfahren die Position des β-Lactamase-Peaks festzustellen. Eine weitere vorteilhafte Verwendung der erfindungsgemäßen Verbindungen liegt in enzymkinetischen Untersuchungen zur Bestimmung von β-Lactamase-Inhibitoren, z. B. Clavulansäure, die die Reaktion einer geeigneten β-Lactamase mit den erfindungsgemäßen Verbindungen ganz oder teilweise verhindern.

## Beispiel 1

7-β-Thien-2-yl-acetamido-3-[(4-nitro-phenyl)-thiomethyl]-3-cephem-4-carbonsäure

2 g p-Nitrothiophenol wurden mit 4,2 g 7-Thien-2-yl-acetamido-cephalosporansäure-Natriumsalz in 120 ml Aceton/Wasser (1/1) bei pH 6.5 bis 7.2 und 64 °C 2 Stunden gerührt. Nach Zugabe von weiteren 0.5 g p-Nitrothiophenol wurde weitere 2 Stunden gerührt. Nach Abkühlen auf Raumtemperatur wurde mit 2N Salzsäure auf pH 7.0 eingestellt, mit Essigester zweimal extrahiert, Essigester-Reste aus der wäßrigen Phase am Rotationsverdampfer entfernt und die wäßrige Phase bei 0 °C mit 2N Salzsäure auf pH 2.0 angesäuert. Es entstand ein hellgelber Niederschlag, der abgesaugt und im Vakuum über Phosphorpentoxid getrocknet wurde : erhalten wurden 3.2 g der Titelverbindung.

NMR (DMSO-$d_6$) :
δ = 6.9-7.5 ppm (Thienyl, 3H)
δ = 4.2 ppm (s, 2H, $CH_2CO$)
δ = 9.0 ppm (d, 1H, CONH)
δ = 5.6 ppm (q, 1H, C-7-H)
δ = 5.1 ppm (d, 1H, C-6-H)
δ = 3.8 ppm (S, 2H, C-2-$CH_2$)
  3.6 (AB, 2H, $CH_2$-S)
δ = 7.5 ppm (4H, p-disubst.-Phenyl)
  8.1

## Beispiel 2

7-β-Thien-2-yl-acetamido-3-[(4-nitro-3-carboxyphenyl)-thiomethyl]-3-cephem-4-carbonsäure

16.7 g 7-Thien-2-yl-acetamido-cephalosporansäure-Natriumsalz wurden in 650 ml Wasser gelöst und unter Stickstoff gerührt. 50 mMol 5-Mercapto-2-nitro-benzoesäure-di Natriumsalz in 100 ml Wasser wurden langsam zugetropft, wobei der pH bei 7.0 gehalten wurde. Bei pH 6.9 bis 7.1 wurde 6 Stunden auf 60 °C erwärmt und anschließend im Kühlschrank belassen. Der pH wurde auf 5.0 gebracht und die Lösung dreimal mit je 200 ml Essigester extrahiert. Die wäßrige Phase wurde von Essigester-Resten befreit, auf 0 °C gekühlt und mit 2N Salzsäure auf pH 2.0 angesäuert. Der Niederschlag wurde über Phosphorpentoxid getrocknet : erhalte wurden 22 g der Titelverbindung. F. : 99-104 °C (Z).

NMR (DMSO-$d_6$) :
δ = 9.0-9.2 ppm (d, 1H, CONH)
δ = 7.2-8.1 ppm (m, arom. Protonen)
δ = 6.8-7.0 ppm (m, 2H, Thienyl 3H + 4H)
δ = 5.6 ppm (q, 1H, C-7-H)
δ = 5.1 ppm (d, 1H, C-6-H)
δ = 4.3 ppm (s, 2H, $CH_2$-S)
δ = 4.8 ppm (s, 2H, $CH_2CO$)
δ = 4.5 ppm (AB, 2H, C-2-$CH_2$)

## Beispiel 3

7-β-Amino-3-[(4-nitro-3-carboxy-phenyl)-thiomethyl]-3-cephem-4-carbonsäure

2.2 g 7-Amino-cephalosporansäure wurden unter Stickstoff in 150 ml Wasser mit Natriumbicarbonat bei pH 7.0 in Lösung gebracht. 10 mMol 5-Mercapto-2-nitro-benzoesäure-di-Natriumsalz in 20 ml Wasser wurden bei pH 6.8 bis 7.3 zugetropft. Es wurde eine Stunde bei Raumtemperatur und 6 Stunden bei 60 °C gerührt, wobei der pH bei 6.8 bis 7.0 gehalten wurde. Es wurde auf Raumtemperatur gekühlt, mit 2N

Salzsäure pH 5.0 gebracht und dreimal mit je 50 ml Essigester extrahiert. Die wäßrige Phase wurde von Essigester-Resten befreit, auf 0 °C gekühlt und auf pH 2.0 angesäuert. Es wurde abgesaugt und getrocknet. Die Ausbeute betrug 2,4 g der Titelverbindung F. : 230 °C (Z).

NMR ($DMSO-d_6$) :
δ = 8.1-7.5 ppm (m, arom. Protonen)
δ = 5.1 ppm (d, 1H, C-7-H)
δ = 4.8 ppm (d, 1H, C-6-H)
δ = 4.3 ppm (d, 2H, $CH_2$-S)
δ = 3.6 ppm (AB, 2H, C-2-$CH_2$)

## Beispiel 4

7-β-Thien-2-yl-acetamido-3-[(4-nitro-3-carboxy-phenyl)-thiomethyl]-3-cephem-4-carbonsäure

822 mg 7-Amino-3-[(4-nitro-3-carboxy-phenyl)-thiomethyl]-3-cephem-4-carbonsäure werden in 200 ml Wasser mit 500 mg Natriumbicarbonat in Lösung gebracht, mit 20 ml Aceton versetzt und im Eisbad auf 0 bis 5 °C gekühlt. Unter Rühren werden 365 mg Thien-2-yl-acetylchlorid in 5 ml Aceton zugetropft. Nach 30 minütigem Rühren werden weitere 50 mg Thien-2-yl-acetylchlorid in 2 ml Aceton zugegeben. Nach 1/2 Stunde wird das Aceton abgezogen und die wäßrige Phase mit 2N Salzsäure auf pH 2.0 angesäuert. Der Niederschlag wird gesammelt, mit Wasser gewaschen und getrocknet : erhalten werden 670 mg der Titelverbindung, in den physikalischen Eigenschaften identisch mit der nach Beispiel 2 erhaltenen Verbindung.

## Patentansprüche

1. Cephalosporine der allgemeinen Formel I

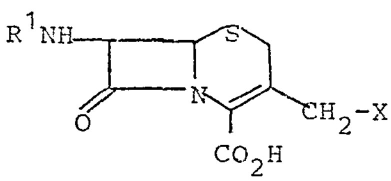

in der $R^1$ Wasserstoff, Formyl, Benzoyl, das substituiert sein kann durch Alkyl mit 1-4 C-Atomen, durch Halogen, durch Nitro, durch Amino oder durch Acetamido, oder ein Rest der Formel

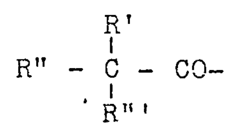

sein kann, in der R′ für Wasserstoff, für Alkyl mit 1-4 C-Atomen, das substituiert ist durch Halogen, durch Cyano, durch Hydroxy, durch ω-Carboxy, oder für gesättigten oder einen ein- oder mehrfach ungesättigten 5- bis 7-gliedrigen carbocyclischen Ring oder für Phenyl und Phenoxy, die substituiert sind durch Alkyl mit 1-4 C-Atomen, durch Hydroxy, durch Alkoxy mit 1-4 C-Atomen, durch Acyloxy mit 1-4 C-Atomen, durch Halogen, durch Carboxy, durch Sulfoxy, durch Amino oder durch Acylamino mit 1-4 C-Atomen oder für einen 5- oder 6-gliedrigen Heteroaryl- oder Heteroarylthio-Rest, der als Heteroatome im Ring Stickstoff, Schwefel oder Sauerstoff enthalten kann, steht, und in der R″ und R‴, die gleich oder verschieden sein können, für Wasserstoff, Hydroxy, Acyloxy mit 1-4 C-Atomen im Acylteil, Amino, Alkylamino mit 1-6 C-Atomen im Alkylteil, Benzylamino, p-Methoxybenzylamino, Benzhydrylamino, Tritylamino, Phenäthylamino, Formamido, Acetylamino, Chloracetylamino, Bromacetylamino, Benzoylamino, tert.-Butoxycarbonylamino, 2,2,2-Trichloräthoxycarbonylamino, 4-Hydroxy-1,5-naphthyridin-3-carbonylamino, 3-Hydroxy-pyridazin-4-carbonylamino, Imidazolidin-2-on-1-yl-carbonylamino, (3-Methylsulfonyl-imidazolidin-2-on-1-yl)-carbonylamino, (4-Äthyl-piperazin-2,3-dion-1-yl)-carbonylamino, Alkoxycarbonyl mit 1-4 C-Atomen im Alkylteil, Halogen, Cyano, Sulfoxy oder Aminosulfonyl stehen, und X steht für eine Gruppe der Formel

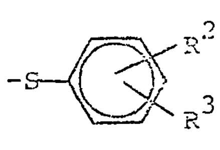

in der $R^2$ für Nitro und $R^3$ für Wasserstoff, Nitro, Halogen, Alkyl mit 1-4 C-Atomen, Cyano, Carboxy, Alkoxycarbonyl mit 1-4 C-Atomen im Alkoxyteil und Aminocarbonyl steht.

2. Cephalosporin gemäß Anspruch 1, dadurch gekennzeichnet, daß R′ in der Bedeutung eines 5- oder 6-gliedrigen Heteroaryl- oder Heteroarylthio-Restes für 2-Furyl, 2-Thienyl, 1-Tetrazolyl, 2- oder 4-Pyridon-1-yl, 3,5-Dichlor-4-pyridon-1-yl, 2-Methyl-1,3,4-thiadiazol-5-yl-thio oder 2-Amino-1,3,4-thiadiazol-5-yl-thio steht.

3. Verfahren zur Herstellung von Cephalosporinen der allgemeinen Formel I gemäß Anspruch 1, dadurch gekennzeichnet, daß man eine Verbindung der Formel III

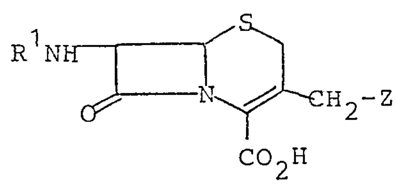

in der Z Formyloxy, Acetoxy, Acetoacetoxy oder Aminocarbonyloxy sein kann, und $R^1$ die vorgenannte Bedeutung hat, mit einer Verbindung der Formel H—X, in der X die vorgenannte Bedeutung hat, umsetzt und im Falle, daß $R^1$ Wasserstoff bedeutet, mit Ameisensäure, mit einer Phenylcarbonsäure, die im Phenylrest durch Alkyl mit 1-4 C-Atomen, Halogen, Nitro, Amino oder Acetamido substituiert sein kann, oder mit einer Carbonsäure der Formel IV

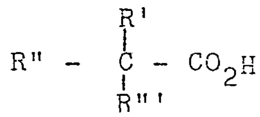 (IV)

in der R′, R″ und R‴ die vorstehend genannten Bedeutungen haben, oder einem reaktionsfähigen Derivat derselben zur Umsetzung bringt.

4. Verfahren nach Anspruch 3, dadurch gekennzeichnet, daß eine Verbindung der Formel H—X in Form ihres Salzes eingesetzt wird.

5. Zubereitungen zum Nachweis von β-Lactamasen, die ein chromogenes Cephalosporin der allgemeinen Formel I gemäß Anspruch 1 in Lösung oder auf einer Trägersubstanz erhalten.

6. Zubereitungen gemäß Anspruch 5, in denen die Trägersubstanz Papier (Teststreifen), einen saugfähigen Kunststoff, ein Dextran oder ein anderes natürliches Polymeres darstellt.

7. Verfahren zur Herstellung von Zubereitungen gemäß Ansprüchen 5 und 6, dadurch gekennzeichnet, daß man ein Cephalosporin der allgemeinen Formel I gemäß Anspruch 1 in einem geeigneten Lösungsmittel löst oder es mit Trägersubstanzen mischt oder es auf diese aufbringt.

8. Verwendung von Cephalosporinen der Formel I gemäß Anspruch 1 zum Nachweis von β-Lactamasen.

9. Verwendung von Cephalosporinen der Formel I gemäß Anspruch 1 zum Nachweis von β-Lactamase-Inhibitoren.

**Claims**

1. Cephalosporins of the general formula I

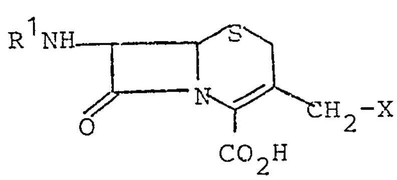

in which $R^1$ is hydrogen, formyl, benzoyl, optionally substituted by alkyl with 1-4 carbon atoms, by halogen, by nitro, by amino or by acetamido, or a radical of the formula

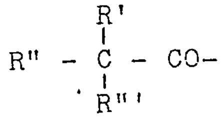

in which R′ is hydrogen, alkyl with 1-4 carbon atoms, optionally substituted by halogen, by cyano, by hydroxy, by ω-carboxy, or wherein R′ is a saturated or a one or several times unsaturated 5- to 7-membered carbocyclic ring or is phenyl and phenoxy, which are substituted by alkyl with 1-4 carbon atoms, by hydroxy, by alkoxy with 1-4 carbon atoms, by acyloxy with 1-4 carbon atoms, by halogen, by

carboxy, by sulfoxy, by amino or by acylamino with 1-4 carbon atoms or is a 5- or 6-membered heteroaryl- or heteroarylthio substituent, which may contain nitrogen, sulfur or oxygen as hetero atoms in the ring, and wherein R″ and R‴, which may be identical or different, are hydrogen, hydroxy, acyloxy with 1-4 carbon atoms in the acyl part, amino, alkylamino with 1-6 carbon atoms in the alkyl part, benzylamino, p-methoxybenzylamino, benzhydrylamino, tritylamino, phenethylamino, formamido, acetylamino, chloracetylamino, bromacetylamino, benzoylamino, tert-butoxycarbonylamino, 2,2,2-trichloroethoxycarbonylamino, 4-hydroxy-1,5-naphthyridin-3-carbonylamino, 3-hydroxy-pyridazin-4-carbonylamino, imidazolidin-2-on-1-yl-carbonylamino, (3-methylsulfonyl-imidazolidin-2-on-1-yl)-carbonylamino, (4-ethylpiperazin-2,3-dion-1-yl)-carbonylamino, alkoxycarbonyl with 1-4 carbon atoms in the alkyl part, halogen, cyano, sulfoxy, or aminosulfonyl, and X is a group of the formula

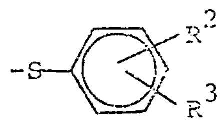

in which $R^2$ means nitro, and $R^3$ is hydrogen, nitro, halogen, alkyl with 1-4 carbon atoms, cyano, carboxy, alkoxycarbonyl with 1-4 carbon atoms in the alkoxy part and aminocarbonyl.

2. Cephalosporin according to claim 1, wherein R′ in the meaning of a 5- or 6-membered heteroaryl- or heteroarylthio substituent is 2-furyl, 2-thienyl, 1-tetrazolyl, 2- or 4-pyridon-1-yl, 3,5-dichloro-4-pyridon-1-yl, 2-methyl-1,3,4-thiadiazol-5-yl-thio or 2-amino-1,3,4-thiadiazol-5-yl-thio.

3. Process for the manufacture of cephalosporins of the general formula I according to claim 1, which comprises reacting a compound of the general formula III

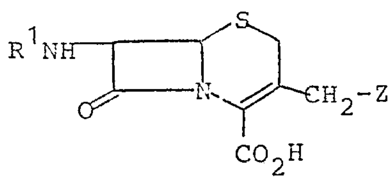

wherein Z means formyloxy, acetoxy, acetoacetoxy or aminocarbonyloxy, and $R^1$ has the above-mentioned meaning, with a compound of the formula H—X, wherein X has the above-mentioned meaning and in the case of $R^1$ being hydrogen, reacting it with formic acid, with a phenylcarboxylic acid, which may be substituted in the phenyl part by alkyl with 1-4 carbon atoms, halogen, nitro, amino, or acetamido, or with a carboxylic acid of the formula IV

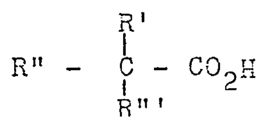

wherein R′, R″ and R‴ have the above-mentioned meanings or a reactive derivative thereof.

4. Process according to claim 3, which comprises using a compound of the formula H—X in the form of its salt.

5. Preparations for the detection of β-lactamases which contain a chromogenic cephalosporin of the formula I according to claim 1 in dissolved form or on a carrier substance.

6. Preparations according to claim 5, wherein the carrier substance is paper (test strip), an adsorbent plastic material, a dextran or other natural polymer.

7. Process for the manufacture of preparations according to claims 5 and 6, which comprises dissolving a cephalosporin of the general formula I according to claim 1 in a suitable solvent or mixing it with carrier substances or applying it to them.

8. Use of cephalosporins of the formula I according to claim 1 for the detection of β-lactamases.

9. Use of cephalosporins of the formula I according to claim 1 for the detection of β-lactamase inhibitors.

**Revendications**

1. Céphalosporine de formule générale I

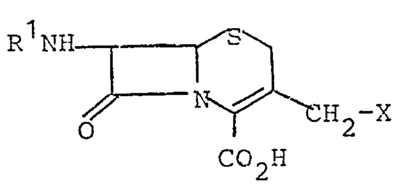

(I)

dans laquelle $R^1$ représente l'hydrogène, un radical formyle benzoyle, qui peut être substitué par un groupe alkyle à 1 à 4 atomes de C, par un halogène, par un groupe nitro, par un groupe amino ou par un groupe acétamido, ou un radical de formule :

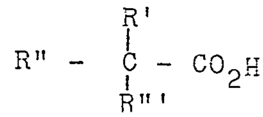

dans laquelle R′ représente l'hydrogène, un groupe alkyle à 1 à 4 atomes de C, qui est substitué par un halogène, par un groupe cyano, par un groupe hydroxy, par un groupe ω-carboxy ou un cycle carboné à 5 à 7 atomes saturé ou mono- ou poly-insaturé, ou un radical phényle ou phénoxy, qui sont substitués par un groupe alkyle à 1 à 4 atomes de C, par un groupe hydroxy, par un groupe alcoxy à 1 à 4 atomes de C, par un groupe acyloxy à 1 à 4 atomes de C, par un halogène, par un groupe carboxy, par un groupe sulfoxy, par un groupe amino ou par un groupe acylamino à 1 à 4 atomes de C, ou un radical hétéroaryle ou hétéroarylthio à 5 ou 6 atomes qui peut contenir comme hétéroatome dans le cycle de l'azote, du soufre ou de l'oxygène, et dans laquelle R″ et R‴, qui peuvent être identiques ou différents, représentent l'hydrogène, un groupe hydroxy acyloxy à 1 à 4 atomes de C dans la partie acyle, amino, alkylamino à 1 à 6 atomes de C dans la partie alkyle, benzylamino, p-méthoxybenzylamino, benzhydrylamino, tritylamino, phénéthylamino, formamido, acétylamino, chloracétylamino, bromacétylamino, benzoylamino, tert-butoxycarbonylamino-2,2,2-trichloroéthoxycarbonylamino, 4-hydroxy-1,5-naphthyridine-3-carbonylamino, 3-hydroxy-pyridazine-4-carbonylamino, imidazolidin-2-one-1-yl-carbonylamino, 3-méthyl-sulfonylimidazolidin-2-one-1-yl-carbonylamino, (4-éthyl-pipérazine-2,3-dione-1-yl)-carbonylamino, alcoxycarbonyle à 1 à 4 atomes de C dans la partie alkyle, halogène, cyano, sulfoxy ou aminosulfonyle et X représente un groupe de formule :

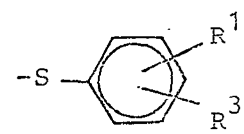

dans laquelle $R^2$ représente un groupe nitro, $R^3$ l'hydrogène, un groupe nitro, un halogène, un groupe alkyle à 1 à 4 atomes de C, un groupe cyano, carboxy, alcoxycarbonyle avec 1 à 4 atomes de C dans la partie alcoxy ou aminocarbonyle.

2. Céphalosporine selon la revendication 1, caractérisée en ce que R′ désigne un radical hétéroaryle ou hétéroarylthio à 5 ou 6 atomes, qui est : 2-furyle-2-thiényle-1-tétrazolyle, 2 ou 4-pyridone-1-yl, 3,5-dichloro-4-pyridone-1-yl, 2-méthyl-1,3,4-thiadiazol-5-yl-thio ou 2-amino-1,3,4-thiadiazol-5-yl-thio.

3. Procédé de préparation de céphalosporines de formule générale I, selon la revendication 1, caractérisé en ce que l'on fait réagir un composé de formule III :

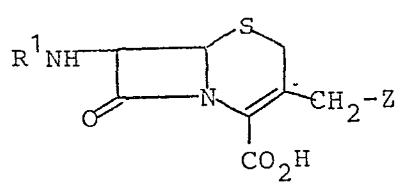

(III)

dans laquelle Z peut être un groupe formyloxy, acétoxy, acétoacétoxy ou aminocarbonyloxy et $R^1$ a la signification indiquée ci-dessus, avec un composé de formule H—X dans laquelle X a la signification indiquée ci-dessus, et, au cas où $R^1$ représente l'hydrogène, avec l'acide formique, avec un acide phénylcarboxylique, qui peut être substitué dans un radical phényle par un groupe alkyle à 1 à 4 atomes de C, par un halogène, par un groupe nitro, amino ou acétamido, ou avec un acide carboxylique de formule IV :

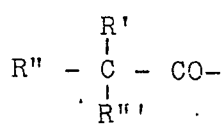

dans laquelle R′, R″ et R‴ ont les significations indiquées ci-dessus, ou avec un dérivé réactif de celui-ci.

4. Procédé selon la revendication 3, caractérisé en ce que l'on utilise un composé de formule H—X sous la forme d'un de ses sels.

5. Préparations pour la détection de β-lactamases qui contiennent une céphalosporine chromogène de formule générale I selon la revendication 1, en solution ou sur une substance support.

6. Préparations selon la revendication 5, dans lesquelles la substance support est du papier (bande pour test), une matière plastique absorbante, un dextranne ou un autre polymère naturel.

7. Procédé de fabrication de préparations selon les revendications 5 et 6, caractérisé en ce que l'on dissout dans un solvant approprié une céphalosporine de formule générale I selon la revendication 1, ou on la mélange avec des substances supports, ou on l'applique sur celles-ci.

8. Utilisation de céphalosporines de formule I selon la revendication 1 pour la détection des β-lactamases.

9. Utilisation de céphalosporines de formule I selon la revendication 1 pour la détection des inhibiteurs des β-lactamases.